# EUROPEAN PATENT SPECIFICATION

(11) **EP 1 594 826 B1**
(45) Date of publication and mention of the grant of the patent: **03.10.2007**
(21) Application number: 04707797.9
(22) Date of filing: 03.02.2004
(51) Int. Cl.: C07C 15/107, C07C 5/27, C07C 2/10

(54) **METHOD OF PREPARING BRANCHED ALKYL AROMATIC HYDROCARBONS USING A PROCESS STREAM FROM A DIMERIZATION UNIT**
VERFAHREN ZUR HERSTELLUNG VERZWEIGTER ALKYLAROMATISCHER KOHLENWASSERSTOFFE UNTER VERWENDUNG EINES VERFAHRENSSTROMS AUS EINER DIMERISIERUNGSEINHEIT
PROCEDE DE PREPARATION D'HYDROCARBURES AROMATIQUES D'ALKYLE RAMIFIE DANS LEQUEL UN COURANT DE TRAITEMENT PROVENANT D'UNE UNITE DE DIMERISATION EST UTILISE

(30) Priority: 05.02.2003 US 445311 P
(43) Date of publication of application: 16.11.2005
(73) Proprietor: SHELL INTERNATIONALE RESEARCH MAATSCHAPPIJ B.V., 2596 HR Den Haag (NL)
(72) Inventor: AYOUB, Paul, Marie, NL-1031 CM Amsterdam (NL); DIRKZWAGER,Hendrik, NL-1031 CM Amsterdam (NL); MURRAY, Brendan, Dermot, Houston, TX 77077 (US); SUMROW, Steven, Colois, Katy, TX 77450 (US)
(86) International application number: PCT/US2004/003010
(87) International publication number: WO 2004/072005

(56) References cited:
- US-A- 3 238 249
- US-A- 3 442 965
- US-A- 4 959 491

## Description

### 1. Field of Invention

The present invention generally relates to systems and methods for preparing alkyl aromatic hydrocarbons. More particularly, embodiments described herein relate to systems and methods for preparing branched alkyl aromatic hydrocarbons.

### 2. Description of Related Art

Alkylated aromatic hydrocarbons are important compounds that may be used in a variety of applications or converted to other chemical compounds (e.g. surfactants, sulfonates). Surfactants may be used in a variety of applications, such as detergents, soaps and oil recovery.

The structural composition of an alkyl aromatic hydrocarbon may influence the properties (e.g., water solubility, biodegradability, cold water detergency) of the surfactant and/or detergent produced from an alkyl aromatic hydrocarbon. For example, water solubility may be affected by linearity of the alkyl group. As the linearity of the alkyl group increases, the hydrophilicity (i.e., affinity for water) of the alkyl aromatic surfactant may decrease. Thus, the water solubility and/or detergency (performance) of the alkyl aromatic surfactant may decrease. Incorporating branches that contain a minimum number of quaternary and/or tertiary carbon atoms into the alkyl portion of the alkyl aromatic surfactant may increase the cold-water solubility and/or detergency of the alkyl aromatic surfactant while maintaining the biodegradability of a detergent. The amount and type of branching of the alkyl group, however, may decrease the biodegradation rate of a surfactant and/or detergent.

Branched alkyl aromatic hydrocarbons are composed of a branched alkyl group coupled to an aromatic group. Branched alkyl groups are composed of a linear alkyl groups with branches extending form the linear alkyl group. Branches of the linear alkyl groups may include one or more aliphatic alkyl groups, a linear alkyl group or combinations thereof. Branches may include methyl, ethyl, propyl or higher alkyl groups. Quaternary and tertiary carbons may be present in a branched the alkyl group. The number of quaternary and tertiary carbons may result from the branching pattern in the alkyl group. As used herein, the phrase "aliphatic quaternary carbon atom" refers to a carbon atom that is not bound to any hydrogen atoms and not attached to an aromatic ring system.

A surfactant with a branched alkyl group including quaternary and/or tertiary carbons may have a lower biodegradation rate than a surfactant with a linear or mono-branched alkyl group. As used herein, "biodegradable" refers to a material that can be chemically altered or broken down by bacteria or other natural agents. For example, in a biodegradation experiment using a porous pot activated sludge treatment, a biodegradation rate of sodium 2-methyl-2-undecyl[¹⁴C]benzensulfonate was greater than a biodegradation rate of sodium 5-methyl-5-undecyl[¹⁴C]benzensalfonate. A detailed description of the experiment is described by Nielsen et al. in "Biodegradation of Coproducts of Commercial Linear Alkylbenzene Sulfonate," Environmental Science and Technology, 1997, 31:3397-3404.

Examples of compositions and process for the manufacture of branched alkyl aromatic hydrocarbons are described in U.S. Patent No. 3,484,498 to Berg, entitled "Process For The Preparation Of Aryl-Substituted Normal Paraffin Hydrocarbons"; U.S. Patent 5,196,624 to Threlkel et al., entitled "Detergent Grade to C₁₀ to C₂₈ Olefins, (C₁₀ to C₂₈ Alkyl)Benzenes and C₁₀ to C₂₈ Alkyl)Benzene Sulfonates and Process for Preparing Same Using A Phosphite Containing Catalyst"; U.S. Patent No. 5,196,625 to Threlkel et al. entitled "Detergent Grade to C₁₀ to C₂₈ Olefins, (C₁₀ to C₂₈ Alkyl) Benzenes and C₁₀ to C₂₈ Alkyl) Benzene Sulfonates and Process for Preparing Same Using A Phosphite Containing Catalyst"; U.S. Patent No. 6,111,158 to Marinangeli et al., entitled "Process For Producing Arylalkanes At Alkylation Conditions Using A Zeolite Having a NES Zeolite Structure Type"; and U.S. Patent No. 6,187,981 to Marinangeli et al., entitled "Process For Producing Arylalkanes And Arylalkane Sulfonates, Compositions Produced Therefrom, and Uses Thereof".

More recently, it has been found that surfactants derived from branched olefins have different, more desirable properties than surfactants derived from linear olefins. For example, surfactants derived from branched olefins have increased water solubility, improved detergency properties and acceptable biodegradable properties compared to surfactants derived from linear olefins. Production of branched olefins from a Fischer-Tropsch process stream may increase an economic value of the stream. In some embodiments, linear olefins may be converted into branched olefins using a dimerization catalyst. The branched olefins may be used to produce surfactants that are more desirable and thus more valuable to the producer of the Fischer-Tropsch stream. In general, Fischer-Tropsch processes tend to produce minor amounts of olefins. Increasing an olefin content (e.g. branched alkyl olefin content) of a Fischer-Tropsch stream may increase the economic value of the stream.

### SUMMARY

In certain embodiments, a feed stream is fed into a dimerization unit that produces dimerized olefins. The produced dimerized olefins may include branched dimerized olefins. A process feed stream entering a dimerization unit is derived, in some embodiments, from a Fischer-Tropsch process. In an embodiment, at least a portion of produced dimerized olefins may be separated from the feed stream. At least a portion of the separated feed stream, in some embodiments, may be introduced into the dimerization unit. Produced dimerized olefins may be used to alkylate aromatic hydrocarbons. After alkylation of the aromatic hydrocarbons, at least a portion of unreacted components from the Alkylation process may be separated from the alkyl aromatic hydrocarbon products.

Process conditions in the dimerization unit may be such that the resulting branched olefins have an average number of branches per olefin molecule of between 0.7 and 2.5. Branched olefins may include, but are not limited to, methyl and/or ethyl branching. The dimerization process may produce branched olefins that include less than 0.5 percent of quaternary carbon atoms. In an embodiment, a feed stream entering the dimerization unit includes alpha-olefins having an average carbon number from 4 to 8. Branched olefins produced from the dimerization of alpha-olefins having an average carbon number from 4 to 8 may have an average carbon number from 8 to 16.

At least a portion of the unreacted components and the produced dimerized olefins may be separated to produce an unreacted hydrocarbon stream and a produced dimerized olefins stream. At least a portion of the unreacted hydrocarbons stream may be recycled to the dimerization unit.

Alkylation of aromatic hydrocarbons with olefins may occur in an alkylation unit. In an embodiment, an olefinic hydrocarbons stream from a dimerization unit and aromatic hydrocarbons may enter an alkylation unit. In the alkylation unit, at least a portion of the aromatic hydrocarbons may be alkylate with at least a portion of the olefins in the hydrocarbon stream to produce alkyl aromatic hydrocarbons. At least a portion of the produced alkyl aromatic hydrocarbons may include a branched alkyl group. At least a portion of the unreacted components of the hydrocarbon stream, at least a portion of unreacted aromatic hydrocarbons and at least a portion of the produced alkyl aromatic hydrocarbons may form an alkylation reaction stream.

At least a portion of the paraffins, unreacted olefins, aromatic hydrocarbons and alkyl aromatic hydrocarbons from the alkylation reaction stream may be separated to produce an unreacted hydrocarbons stream and an alkyl aromatic hydrocarbons product stream. The unreacted hydrocarbons stream may be further separated, in some embodiments, to form a paraffins and unreacted olefins stream and an aromatic hydrocarbons stream. At least a portion of the unreacted aromatic hydrocarbons stream may be recycled to the alkylation unit.

In certain embodiments, at least a portion of the alkyl aromatic hydrocarbons product stream may be sulfonated to form alkyl aromatic sulfonates. In some embodiments, the alkyl aromatic sulfonates may include branched alkyl groups.

### BRIEF DESCRIPTION OF THE DRAWINGS

Advantages of the present invention will become apparent to those skilled in the art with the benefit of the following detailed description of embodiments and upon reference to the accompanying drawings, in which:
FIG. 1 depicts a schematic diagram of an embodiment of a system for producing branched alkyl aromatic hydrocarbons using a dimerization unit.
FIG. 2 depicts a schematic diagram of an embodiment of a separation unit to separate produced dimerized olefins from a reaction mixture.

While the invention is susceptible to various modifications and alternative forms, specific embodiments thereof are shown by way of example in the drawing and will herein be described in detail. It should be understood that the drawings and detailed description thereto are not intended to limit the invention to the particular form disclosed, but on the contrary, the intention is to cover all modifications, equivalents, and alternatives falling within the spirit and scope of the present invention as defined by the appended claims.

### DETAILED DESCRIPTION OF EMBODIMENTS

Hydrocarbon products may be synthesized from synthesis gas (i.e., a mixture of hydrogen and carbon monoxide) using a Fischer-Tropsch process. Synthesis gas may be derived from coal or by reforming of natural gas. The Fischer-Tropsch process catalytically converts synthesis gas into a mixture of products that includes primarily saturated hydrocarbons, a certain amount of olefins and a minor amount of oxygen containing products. The products from a Fischer-Tropsch process may be used for the production of fuels (e.g., gasoline, diesel oil), lubricating oils and waxes. Fuels and other products produced by a Fischer-Tropsch process generally have low levels of sulfur, nitrogen and/or metals and contain little or no cyclic compounds (e.g., (aromatics, naphthalenes).

Fischer-Tropsch process streams may be used to prepare economically valuable commodity products. For example, linear olefins are commodity products that are useful for production of surfactants. Using a portion of the process stream to produce linear olefins may increase the economic value of a Fischer-Tropsch process stream.

A hydrocarbon feed stream may be obtained from a Fischer-Tropsch process or from an ethylene oligomerization process. Fischer-Tropsch catalysts and reaction conditions may be selected to provide a particular mix of products in a reaction product stream. For example, a Fischer-Tropsch catalyst and reaction conditions may be selected to increase the amount of olefins and decrease the amount of paraffins and oxygenates in the stream. Alternatively, the catalyst and reaction conditions may be selected to increase an amount of paraffins and decrease an amount of olefins and oxygenates in the stream. Catalysts and/or combinations of catalyst that favor the manufacture of desired hydrocarbon species in a Fischer-Tropsch process are generally known.

While reference is made to a Fischer-Tropsch stream, it is to be understood that any stream, made by any process, that includes olefins and saturated hydrocarbons may be a suitable feedstock for the processes disclosed herein. Many Fischer-Tropsch streams may contain from 5 percent to 99 percent olefins, the remainder being saturated hydrocarbons and other compounds.

In some embodiments, feed streams containing olefins and paraffins are obtained through cracking of paraffin wax or the oligomerization of olefins. Commercial olefin products manufactured by ethylene oligomerization are marketed in the United States by Chevron Chemical Company, Shell Chemical Company (under the trademark NEODENE), and by British Petroleum. Cracking of paraffin wax to produce alpha-olefin and paraffin feed streams is described in U.S. Patent No. 4,579,986 to Sie, entitled "Process For The Preparation Of Hydrocarbons" and U.S. Patent Application Serial No. 10/153,955 to Ansorge et al., entitled "Process For The Preparation of linear Olefins and Use Thereof To Prepare Linear Alcohols". Specific procedures for preparing linear olefins from ethylene are described in U.S. Patent No. 3,676,523 to Mason, entitled "Alpha-Olefin Production"; U.S. Patent No. 3,686,351 to Mason, entitled "Alpha-Olefin Production; "U.S. Patent No. 3,737,475 to Mason, entitled "Alpha-Olefin Production" and U. S. Patent No. 4,020,121 to Kister et al., entitled "Oligomerization Reaction System." Most of the above-mentioned processes produce alpha-olefins. Higher linear internal olefins may be commercially produced, for example, by the chlorination-dehydrochlorination of paraffins, by paraffin dehydrogenation, or by isomerization of alpha-olefins.

In an embodiment, a feed stream is processed to produce a hydrocarbon stream that includes branched olefins. Branched olefins may be used to alkylate aromatic hydrocarbons to produce branched alkyl aromatic hydrocarbons. The feed stream may have a paraffin content range between 50 percent by weight and 90 percent by weight of the feed stream. In certain embodiments, a feed stream may have a paraffin content greater than 90 percent by weight paraffins. The feed stream may also include olefins. The olefin content of the feed stream may be between 10 percent by weight and 50 percent by weight. In other embodiments, a feed stream may have an olefin content greater than 90 percent by weight olefins. The composition of the feed stream may include hydrocarbons having an average carbon number greater than 4. The average carbon number of the hydrocarbons may range from 4 to 6. In certain embodiments, an average carbon number of the hydrocarbons may range from 4 to 8. A feed stream may include minor amounts of hydrocarbons having a carbon number that is higher or lower then the desired carbon number range. In some embodiments, a feed stream may be derived from distillation of a process stream that includes a broader range of carbon numbers.

In an embodiment, a feed stream for a dimerization unit includes mono-olefins and/or paraffins. The mono-olefins may be of a linear or branched structure. The mono-olefins may have an alpha or internal double bond. The feed stream may include olefins in which 50 percent or more of the olefin molecules present may be alpha-olefins of a linear (straight chain) carbon skeletal structure. In certain embodiments, at least about 70 percent of the olefins are alpha-olefins of a linear carbon skeletal structure. A hydrocarbon stream in which greater than 70 percent of all of the olefin molecules are alpha-olefins of a linear carbon skeletal structure may be used in certain embodiments of alkylation of aromatic hydrocarbons. Such a stream may be derived from a Fischer-Tropsch process. In some embodiments, a feed stream includes olefins in which at least about 50 percent of the olefin molecules present are internal olefins.

Branched chain olefins may be used as a feed source for an aromatic alkylation reaction. An aromatic alkylation reaction uses olefins in an incoming stream to alkylate aromatic compounds. Aromatic compounds (e.g., aromatic hydrocarbons) may include benzene or substituted benzene. In an embodiment, alkyl-substituted benzene is used as a substituted benzene in the process. Alkyl-substituted benzenes may be mono- and/or polysubstituted lower alkyl benzenes. The alkyl substituent of an alkyl substituted benzene may have a carbon number ranging from 1 to 5. In an embodiment, a carbon number of the alkyl substituent may range from 1 to 2. Suitable examples of aromatic compounds include, but are not limited to, benzene, toluene, xylenes, ethylbenzene, cumene, n-propylbenzene and other mono- and poly-lower alkylbenzenes. In some embodiments, a single aromatic compound or a mixture of two or more aromatic compounds may be used as feed source. The aromatic hydrocarbons may be fed into the reactor directly or mixed in a suitable non-reactive organic solvent prior to addition to the alkylation unit.

A Fischer-Tropsch feed stream may contain olefins and paraffins of low carbon number (e.g., 4, 5, 6, 7, 8). Typically, a low carbon number feed stream may be sold as fuel, sent to waste and/or recycled to other processing units. The low carbon number of the paraffins and olefins makes the stream less useful for the production of detergents. Typically detergents are made from olefins having a carbon number greater than 8. Conversion of the olefins in the feed stream to branched olefins with higher average carbon number (e.g., 8 to 16) may result in a more commercially valuable use of a low carbon number feed stream (e.g., a feed stream that may be processed to produce detergents and/or surfactants). The amount and type of branching of the alkyl group may increase the value of the feed stream. Processes to convert low carbon number olefins to higher carbon number olefins are described herein.

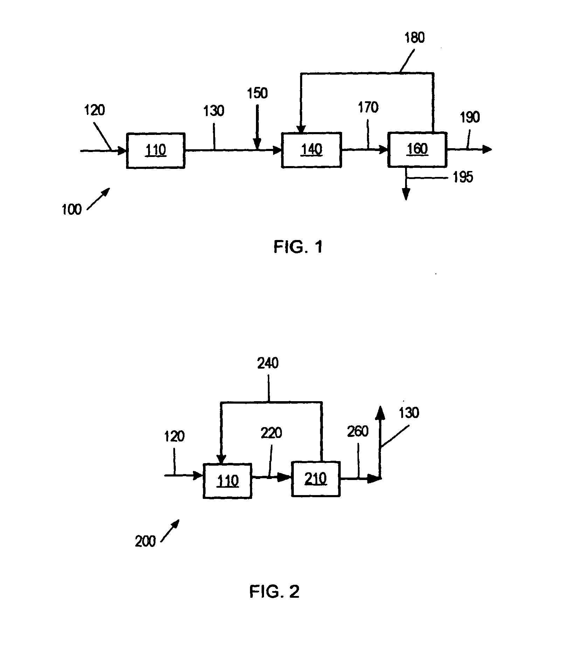

Referring to System 100 depicted in FIG. 1, a first hydrocarbon stream, including olefins and paraffins may be introduced into dimerization unit 110 via first conduit 120. In certain embodiments, hydrocarbons in the first hydrocarbon stream may have an average carbon number from 4 to 8. In other embodiments, hydrocarbons in the first hydrocarbon stream may have an average carbon number from 4 to 6. The first hydrocarbon stream may, in some embodiments, be derived from a Fischer-Tropsch process. In dimerization unit 110, at least a portion of the olefins may be dimerized. At least a portion of the dimerized olefins exit dimerization unit 110 as a second hydrocarbon stream via second conduit 130. Depending on the choice of catalyst, the resulting dimerized olefins may be branched. The branches of the olefin produced in dimerization unit 110 may include methyl, ethyl and/or longer carbon chains. In an embodiment, dimerized olefins may contain greater than about 50 percent methyl branches. In some embodiments, resulting dimerized olefins may contain greater than about 90 percent methyl branches.

The dimerization catalyst used in dimerization unit 110 may be a homogeneous or heterogeneous catalyst. In certain embodiments, a dimerization catalyst used in dimerization unit 110 may be a catalyst that includes oxides of Group III, Group IVA, Group IVB, Group VIIIA, or combinations thereof. Examples of such oxides include, but are not limited to, nickel oxide, silicon dioxide, titanium dioxide, aluminum oxide or zirconium dioxide. The catalyst may include an amorphous nickel oxide (NiO) present as a dispersed substantial monolayer on the surfaces of a silica (SiO₂) support. A surface of silica support may also include minor amounts of an oxide of aluminum, gallium or indium such that the ratio of nickel oxide to metal oxide present in the catalyst is within the range of from 4:1 to 100:1. The dimerization catalyst may be prepared by precipitating a water insoluble nickel salt onto the surface of a silica support that has been impregnated with the metal oxide. Alternatively, a dimerization catalyst may be prepared by precipitating a water insoluble nickel salt onto a silicaalumina support, which has been dealuminized such that the resulting nickel oxide/alumina ratio falls within the range of from 4:1 to 100:1. The catalyst may be activated by calcination in the presence of oxygen at a temperature within a range of from 300 °C to 700 °C or, in some embodiments, a temperature range from 500 °C to 600 °C.

A silica support surface area may be within the range of from 100 m²/g to 450 m²/g. In an embodiment, a silica surface area may be within the range from 200 m²/g to 400 m²/g. A nickel oxide content dispersed in the silica support may range from 7 percent to 70 percent by weight. In certain embodiments, a nickel oxide content may be from 20 percent to 50 percent by weight, depending on the surface area of the particular support utilized in preparing a catalyst. For a silica support having a surface area of 300 m²/g, a nickel oxide content may, in some embodiments, ranges from 21 percent to 35 percent by weight. A nickel oxide content may, in other embodiments, be 28 percent by weight.

The silica support may be in dry granular form or in a hydrogel form prior to precipitation of the nickel oxide precursor compound on the surfaces thereof. Silica hydrogel may be prepared by mixing a water-soluble silicate (e.g., a sodium or potassium silicate) with a mineral acid then washed with water to remove water-soluble ions. The resulting silica hydrogel may be partially or completely dried.

A nickel oxide precursor may include a water-insoluble nickel salt, such as nickel carbonate, nickel phosphate, nickel nitrate or nickel hydroxide. The water-insoluble nickel salt may be generated in-situ by forming an aqueous mixture of silica gel and a water-soluble nickel salt. The nickel salt may include, but is not limited to, nickel nitrate, nickel sulfonate, nickel carbonylate, nickel halide. A base may be added to the aqueous mixture to induce precipitation of a water-insoluble nickel salt. The water-insoluble nickel salt, which may be formed, may be precipitated in finely divided form within the interstices and on the surface of the silica support. The treated silica support may then be recovered, washed several times and dried.

A second component in the dimerization catalyst may be a trivalent metal oxide. The trivalent metal oxides may include, but are not limited to, aluminum, gallium and indium or combinations thereof. Although, a nickel oxide and/or silica catalyst described above may be active for olefin dimerization, deactivation may occur quickly. Deactivation may be a consequence of a formation of large oligomers that remain attached to the surface and act as coke precursors. The presence of a small amount of the trivalent metal oxide within the catalyst yields acid sites. Acid sites may promote catalytic activity without promoting unwanted and/or excessive oligomer formation.

The trivalent metal oxide may be incorporated into the silica support by any suitable technique (e.g., the precipitation method described above or by direct impregnation in the form of a water-soluble salt). In an embodiment, a trivalent metal oxide may be impregnated into the silica support as an aqueous solution by the addition of a water-soluble salt. The water-soluble metal salt may include, but is not limited to, metal nitrates, metal chlorides or metal sulfates. Once impregnated with a metal salt, the silica support may be dried and calcinated to reduce the metal salt to an oxide form. The resulting silica support activated with the trivalent oxide may then be further treated to incorporate the nickel oxide layer onto the silica-trivalent metal oxide support.

In some embodiment, a silica-trivalent metal oxide support may include silica/alumina, silica/gallia or silica/india gel. In certain embodiments, content of a metal oxide (e.g., alumina) present in the support may be low in comparison with the content of nickel oxide. Therefore, dealuminization of the silica/alumina gel of relatively high alumina content (e.g., above 5 percent by weight) may be necessary to reduce the content of alumina. Dealuminization may be accomplished by techniques known in the art (e.g., extraction of the aluminum with an organic or inorganic acid). Organic or inorganic acids may include, but are not limited to, nitric acid, sulfuric acid, hydrochloric acid, chloroacetic acid or ethylene diamine tetraacetic acid. Extraction may be accomplished by adding an acid to an aqueous dispersion of the alumino silicate followed by stirring, decantation and washing with water. The process may be repeated one or more times until a desired alumina content is achieved. The solids may then be dried, calcined and further treated as described above to incorporate the nickel oxide layer onto the silica/alumina support.

The content of trivalent metal oxide with respect to the content of the nickel oxide present in the silica support may be significant, in some embodiments, in order to achieve improved results in terms of dimer yield and minimum average methyl branching present in the dimer product. In certain embodiments, when the content of trivalent metal oxide is too low, e.g., above a nickel oxide to trivalent metal oxide ratio of about 100 to 1, then the yield of dimer decreases and the catalyst may tend to deactivate quickly. In certain embodiments, when the content of trivalent metal oxide is too high, (e.g., below a nickel oxide to trivalent metal oxide ratio of about 4 to 1) then the yield of dimer may tend to decrease and the average content of methyl branching in the dimer product may tend to increase. In certain embodiments, content of a trivalent metal oxide may be such that the ratio of nickel oxide to trivalent metal oxide falls within the range of from 4:1 to 30:1. In other embodiments, content of a trivalent metal oxide may be such that the ratio of nickel oxide to trivalent metal oxide is between 5:1 and 20:1. In certain embodiments, a ratio of nickel oxide to trivalent metal oxide may be between 8:1 and 15:1.

In certain embodiments, a dimerization catalyst may contain from 21 percent to 35 percent by weight of nickel oxide and 1 percent to 5 percent by weight of trivalent metal oxide, based on the total weight of nickel oxide, trivalent metal oxide and silica. In certain embodiments, a dimerization catalyst may include from 1.5 percent to 4 percent by weight trivalent metal oxide based on the total weight of nickel oxide, trivalent metal oxide and silica.

The preparation of dimerization catalysts are described in U.S. Patent 5,849, 972 to Vicari et al., entitled "Oligomerization Of Olefms To Highly Linear Oligomers, and Catalyst For This Purpose", and U.S. Patent, 5,169,824 to Saleh et al., entitled "Catalyst Comprising Amorphous NiO On Silica/Alumina Support."

Conversion of olefins in a first hydrocarbon feed stream to dimers in dimerization unit 110, may be carried out as a batch, continuous (e.g. using a fixed bed), semi-batch or multi-step process. In a batch process, the dimerization catalyst may be slurried with the first hydrocarbon feed stream. Temperature for the dimerization reaction may range from 120 °C to 200 °C. In an embodiment, reaction temperatures may range from 150 °C to 165 °C. Reaction temperatures may be controlled with evaporative cooling, e.g., the evaporation of lighter hydrocarbon fractions from the reaction mixture controls the reaction temperature.

Produced dimerized olefins may exit the dimerization unit in a second hydrocarbon stream via second conduit 130. The second hydrocarbon stream may be transported to other processing units (e.g., a separation unit, an alkylation unit, a hydroformylation unit). In some embodiments, the second hydrocarbon stream may include olefins with an average carbon number from 8 to 16. In other embodiments, the second hydrocarbon stream may include olefins with an average carbon number from 8 to 12. The second hydrocarbon stream may include, in some embodiments, an olefin content of greater than 50 percent by weight.

Branched olefins produced in dimerization unit 110 may include methyl, ethyl and/or longer carbon chain branches. Hydrogen Nuclear Magnetic Resonance (¹H NMR) analysis of the isomerized olefin composition may be performed. Branched olefins may include quaternary and/or tertiary aliphatic carbons. In certain embodiments, amounts of quaternary and/or tertiary aliphatic carbons produced in a dimerization unit may be minimized. ¹H NMR analysis of the olefins may indicate the extent of isomerization of the olefins in the hydrocarbon stream.

The presence of quaternary carbon atoms may be determined using carbon 13 (¹³C) NMR techniques. The type of branching (e.g., methyl, ethyl, propyl or larger groups) may be determined by hydrogenation of the olefin mixtures and then ¹³C NMR analysis of the hydrogenated olefin solution.

In an embodiment, an average number of branches per olefin molecule present in the branched olefin composition is between 0.1 and 2.5. In other embodiments, an average number of branches per olefin molecule present in the branched olefin composition is between 0.7 and 2.5. In certain embodiments, when the feed stream contains greater than 70 percent linear olefins, an average number of branches per olefin molecule present in the branched olefin composition is between 0.2 and 1.5. The degree of branching in the product may be controlled by controlling process conditions used in the dimerization unit. For example, high reaction temperatures and lower feed rates may result in a higher degree of branching. Methyl branches may represent between 20 percent and 99 percent of the total number of branches present in the olefin molecules. In some embodiments, methyl branches may represent greater than 50 percent of the total number of branches in the olefin molecules. The number of ethyl branches in the olefin molecules may represent, in certain embodiments, less than 30 percent of the total number of branches. In other embodiments, a number of ethyl branches, if present, may be between 0.1 percent and 2 percent of the total number of branches. Branches other than methyl or ethyl, if present, may be less than 10 percent of the total number of branches.

The number of aliphatic quaternary and/or tertiary carbon atoms present in the branched olefin composition may be less than about 2 percent of the carbon atoms present. In an embodiment, an aliphatic quaternary and/or tertiary carbons present are less than about 1 percent of the carbon atoms present. For applications where biodegradability is important, the number of aliphatic quaternary carbon atoms may be less than 0.5 percent of the carbon atoms present. In an embodiment, a number of aliphatic quaternary and/or tertiary carbon atoms is less than 0.3 percent of the carbon atoms present. In other embodiments, a number of aliphatic quaternary carbon atoms present in the branched olefin composition is between 0.01 percent and 0.3 percent of the aliphatic carbon atoms present.

The produced dimerized olefins may be separated, if desired, from the reaction mixture through techniques known in the art (e.g., fractional distillation). In an embodiment, at least a portion of a second hydrocarbon stream may exit dimerization unit 110 and enter separation unit 210 via conduit 220 as depicted in FIG. 2. The reaction mixture may be separated into a produced dimerized olefins stream and a paraffins and unreacted olefins stream through fractional distillation in separation unit 210. As used herein "fractional distillation" refers to the distillation of liquids and subsequent collection of fractions of liquids determined by boiling point. The paraffins and unreacted olefins stream may contain hydrocarbons with a carbon number less than 8. At least a portion of the paraffins and unreacted olefins stream may be introduced into dimerization unit 110 via conduit 240. The produced dimerized olefins stream may exit separation unit 210 and be introduced into second conduit 130 via conduit 260.

In certain embodiments, dimerization unit 110 may have several points of entry to accommodate process streams which may vary in composition. The process streams may be from other processing units and/or storage units. Examples of process streams include, but are not limited to, a diluent hydrocarbon stream, and/or other hydrocarbon streams that include olefins and paraffins derived from other processes. As used herein "entry into the dimerization unit" refers to entry of process streams into the dimerization unit through one or more entry points.

At least a portion of the produced dimerized olefins stream may be introduced into alkylation unit 140 via second conduit 130. Alkylation of aromatic hydrocarbons by at least a portion of branched olefins produced in dimerization unit 110 may be conducted using various types of reactors. In certain embodiments, an alkylation process may be carried out in a batch wise fashion by adding the catalyst and aromatic hydrocarbons to a reactor, heating the mixture to a reaction temperature, and then adding the olefinic or aromatic hydrocarbons to the heated mixture.

Alkylation unit 140 may have several points of entry to accommodate the entry of additional process streams. As used herein, "stream entering into the alkylation unit" refers to the entry into the alkylation unit of process streams through one or more entry points. Examples of such process streams include, but are not limited to, streams from dimerization unit 110, a diluent hydrocarbon stream, gases and/or other hydrocarbon streams that include olefms and paraffins derived from other processes.

In an embodiment, at least a portion of the olefins in the second hydrocarbon stream produced by dimerization unit 110 is contacted in alkylation unit 140 with aromatic hydrocarbons (e.g., benzene) using a variety of alkylating conditions. At least a portion of the resulting alkyl aromatic hydrocarbons are monoalkylated aromatic hydrocarbons having a branched alkyl group. Minimization of other alkylation products, such as dialkylation and higher alkylation products, may be controlled by process conditions (e.g., molar ratio, reaction temperatures, catalyst type and reactant concentrations) in alkylation unit 140.

In an embodiment, alkylation conditions for alkylation of aromatic hydrocarbons in alkylation unit 140 may include the use of a Friedel-Crafts catalyst type. The Friedel-Crafts catalyst may be an acidic inorganic material. Examples of acidic inorganic materials include, but are not limited to, mineral acids such as sulfuric acid containing less than about 10 percent water, hydrofluoric acid containing less than about 10 percent water, liquefied anhydrous hydrogen fluoride, and/or other inorganic materials used in combination with hydrofluoric acid. Other inorganic materials may include, but are not limited to, Lewis acids such as anhydrous aluminum chloride, anhydrous aluminum bromide and boron trifluoride.

In certain embodiments, an Alkylation catalyst may be a molecular sieve such as ITQ-21 in the acidic form. The synthesis and structures of molecular sieve catalysts are described by Corma, et al. in "A large-cavity zeolite with wide pore windows and potential as an oil refining catalyst", Nature, 2002, 418:514.

In certain embodiments, an alkylation catalyst used in alkylation unit 140 is based on a zeolite catalyst that may or may not be modified with a metal or metal compound. Zeolite alkylation catalysts are described in U.S. Patent Application Serial No. 10/075,318 entitled "A Process For Preparing (Branched-Alkyl) Arylsulfonates and (Branched-Alkyl) Arylsulfonate Composition," U.S. Patent No. 6,111,158 to Marinangeli et al. entitled "Process For Producing Arylalkanes At Alkylation Conditions Using A Zeolite Having a NES Zeolite Structure Type" and U.S. Patent No. 5,041,402 to Schoennagel et al., entitled "Thermally Stable Noble Metal-Containing Zeolite Catalyst." A zeolite catalyst may have at least one channel with a crystallographic free channel diameter ranging from greater than 4 Å to less than 9 Å. With the understanding that when the pores have an elliptical shape, the larger pore size dimension is the dimension to be considered. In an embodiment, a pore size dimensions may range between 5.5 Å to 7 Å. Pore size dimensions of zeolites are described in W. M. Meier et al., "Atlas of Zeolite Structure Types," Fourth revised edition, 1996, Elsevier.

In alkylation unit 140, at least a portion of the olefins in the second hydrocarbon stream and at least a portion of the aromatic hydrocarbons may be reacted under alkylation conditions in the presence of the alkylation catalyst. Reaction temperatures for the alkylation reaction may range between greater than 30 °C and less than 300 °C. In certain embodiments, reaction temperatures may range between greater than 100 °C to less than 250 °C. An alkylation reaction may be conducted in at least a partial liquid phase, in an all-liquid phase or at supercritical conditions. In certain embodiments, pressures in the alkylation unit are sufficient to maintain reactants in the liquid phase. The pressure used in alkylation unit 140 may depend upon the identity of olefin, the identity of the aromatic hydrocarbon and/or the temperature of the reaction. Pressures in alkylation unit 140 may range between 3 atmospheres and 70 atmospheres (304 kPa-7095 kPa). In certain embodiments, operating pressure range between 20 atmospheres and 35 atmospheres (2025-3545 kPa).

Alkylation conditions in alkylation unit 140 may be maintained to minimize skeletal isomerization of the branched olefins. Alkylation conditions may also be maintained to produce alkyl aromatic hydrocarbons with an alkyl group that corresponds to the branching of the olefins produced in dimerization unit 110. "Skeletal isomerization during alkylation," as used herein, is an isomerization that occurs during alkylation that changes the position of the branching in the olefin or alkyl aromatic hydrocarbon product. Accordingly, alkylation conditions may be set such that the number of quaternary aliphatic carbon atoms in the olefin and the alkyl aromatic hydrocarbon product may remain unchanged during the alkylation reaction.

A general class of branched alkyl aromatic compounds produced in alkylation unit 140 may be characterized by the chemical formula R-Y, in which Y represents an aromatic hydrocarbyl radical (e.g., a phenyl radical) and R represents a radical derived from an olefin produced in dimerization unit 110. The olefin may have a carbon number in the range from 7 to 16. In certain embodiments, an olefin carbon number may range from 10 to 16. An olefin carbon number may, in other embodiments, range from 10 to 13. R may be a branched alkyl radical. Branches on a branched alkyl radical may include, but are not limited to, methyl, ethyl and/or longer carbon chains. The average number of branches per alkyl molecule present in the alkyl composition may be equal to the number of branches in the olefin produced in dimerization unit 110 (e.g., between 0.1 and 2.0).

The stoichiometry of the alkylation reaction requires only one mole of aromatic hydrocarbon compound per mole of total mono-olefins. Using 1:1 molar conditions, however, tends to produce mixtures of olefin oligomers and/or polymers, monoalkyl aromatic hydrocarbons, dialkyl-, trialkyl- and possibly highly polyalkylated aromatic hydrocarbons or unreacted olefins. It is desired, however, to have the molar ratio as close to 1:1 as possible to maximize utilization of the aromatic hydrocarbon and to minimize recycle of unreacted aromatic hydrocarbons or unreacted olefins. The molar proportion of aromatic hydrocarbon to total mono-olefin may influence both conversion and selectivity of the alkylation reaction. In certain embodiments, a molar ratio of total aromatic hydrocarbon per mole of total mono-olefins may be set between about 3:1 and about 100:1 to maximize formation of monoalkyl aromatic hydrocarbon. In some embodiments, an aromatic hydrocarbon to olefin ratio may be from 5:1 to 50:1. In other embodiments, an aromatic hydrocarbon to olefin ratio may be from 5:1 to 35:1. In certain embodiments, an aromatic hydrocarbon to olefin ratio may be from 8:1 to 30:1.

In certain embodiments, it may be desirable to adjust the olefin and paraffin concentration depending on the source of the olefin stream. A paraffinic stream may be added to a process stream that contains greater than 50 percent mono-olefins upstream of an alkylation unit to produce a process stream that is less than 50 percent mono-olefins. In some embodiments, a process stream containing 20 percent linear olefins and 80 percent paraffins may be added to a process stream containing primarily branched olefins upstream of an alkylation unit to form a combined stream. Alkylation of aromatic hydrocarbons with the combined stream may result in a mixed stream containing branched and linear alkyl aromatic hydrocarbons.

At least a portion of a third hydrocarbon stream may be introduced into second conduit 130 via third conduit 150 to produce a combined hydrocarbon stream. The combined stream may enter alkylation unit 140. At least a portion of olefins in the combined stream may alkylate the aromatic hydrocarbons in alkylation unit 140 to produce an alkylation reaction mixture stream. In an embodiment, an olefin content of a third hydrocarbon stream ranges between about 1 percent and about 99 percent relative to the total hydrocarbon content. In certain embodiments, an olefin content of a third hydrocarbon stream may be between about 5 percent and about 15 percent relative to the total hydrocarbon content. In other embodiments, an olefin content of a third hydrocarbon stream may be greater than 80 percent relative to the total hydrocarbon stream.

In some embodiments, the third hydrocarbon stream may include linear olefins. The addition of a stream that includes linear olefins downstream from the dimerization unit allows the creation of an alkylation feed stream that includes a mixture of linear and branched olefins. By introducing a stream including branched and linear olefins into alkylation unit 140, a mixture of branched and linear alkyl aromatic products may be obtained. Varying the amount of linear olefins added to the alkylation feed stream may control the ratio of linear to branched alkyl aromatic products. A mixture of branched and linear alkyl aromatic hydrocarbons may have improved properties when converted to alkyl aromatic surfactants. Examples of improved properties include, but are not limited to, low skin and eye irritation, foaming properties, biodegradability, cold-water solubility and cold-water detergency. Applications for these surfactants include, but are not limited to, personal care products, household and industrial laundry products, hand dishwashing products, machine lubricant additives and lubricating oil formulations.

The alkylation reaction mixture stream may enter separator 160 via fourth conduit 170. At least two streams, an unreacted hydrocarbons stream and an alkyl aromatic hydrocarbons stream may be produced, in separator 160. The unreacted hydrocarbons stream may be separated into an aromatic hydrocarbons stream and a paraffins and unreacted olefins stream using techniques known in the art (e.g., distillation, solid/liquid separation, absorption, solvent extraction). The separated aromatic hydrocarbons stream may exit separator 160 and be recycled to alkylation unit 140 via fifth conduit 180. The alkyl aromatic hydrocarbons product stream may exit separator 160 and be transported via sixth conduit 190 to be stored on site, sold commercially, transported off-site, and/or utilized in other processing units. At least a portion of the paraffins and unreacted olefins stream may exit separator 160 via seventh conduit 195 and be combined with other process streams, sent to other processing units and/or be stored on site. In certain embodiments, the paraffins and unreacted olefins stream may be further separated into a hydrocarbons stream including paraffins and unreacted olefins with a carbon number less than 8. The hydrocarbon stream including paraffins and unreacted olefins with a carbon number less than 8 may be introduced upstream of and/or into dimerization unit 110.

The alkyl aromatic hydrocarbon product stream produced may be sulfonated in a sulfonation unit to form alkyl aromatic sulfonates. In certain embodiments, alkyl aromatic hydrocarbons may contain branched alkyl groups. Sulfonation of at least a portion of the aromatic hydrocarbons in the alkyl aromatic hydrocarbon product stream may be performed by any method of sulfonation known in the art. Examples of such methods include sulfonation using sulfuric acid, chlorosulfonic acid, oleum or sulfur trioxide. Details of a sulfonation method involving an air/sulfur trioxide mixture are described in U.S. Patent No. 3,427,342 to Brooks et al., entitled "Continuous Sulfonation Process." In an embodiment, a sulfur trioxide to alkyl aromatic product molar ratio used for sulfonation is 1.03.

After the sulfonation reaction is complete, the sulfonation reaction mixture may be aged for about thirty minutes and then hydrolyzed with approximately 1% water. The resulting acid mixture may be neutralized with a base to produce a salt of the branched alkyl aromatic hydrocarbon sulfonate. Suitable neutralization bases may be hydroxides of alkali metals and alkaline earth metals, and ammonium hydroxides, which provide the cation of the sulfonate salts.

A general class of branched alkyl aromatic hydrocarbon sulfonates may be characterized by a chemical formula (R-A'-SO₃)*ₙ*M. R represents a radical derived from the branched olefins, having an average carbon number in the range from 4 to 16. In an embodiment, an average carbon number ranges from 7 to 16. In another embodiment, an average carbon number ranges from 10 to 13. A' may represent a divalent aromatic hydrocarbyl radical, (e.g. a phenyl radical). M may be a cation selected from an alkali metal ion, an alkaline earth metal ion, an ammonium ion, and/or mixtures thereof and n may be a number depending on the valence of the cation(s) M, such that the total electrical charge of the complex is zero. In an embodiment, M may be sodium, magnesium or potassium ions. Magnesium and potassium ions may promote water solubility and overall performance of the alkyl aromatic hydrocarbon sulfonate. Examples of ammonium ions may include, but are not limited to, monoethanol amine, diethanol amine and triethanol amine. In certain embodiments, an ammonium ion may be represented by NH₄⁺. The resulting alkyl aromatic hydrocarbon sulfonate salt may be stored and/or sold as a solution (e.g. 30% aqueous solution), slurry (e.g., 60% aqueous slurry) and/or flakes (e.g., 90% dried flakes).

Branched alkyl aromatic sulfonates may be used in a wide variety of applications. An example of an application includes detergent formulations. Detergent formulations include, but are not limited to, granular laundry detergent formulation, liquid laundry detergent formulations, liquid dishwashing detergent formulations and miscellaneous formulations. Examples of miscellaneous formulations include, but are not limited to, general purpose cleaning agents, liquid soaps, shampoos and liquid scouring agents.

### Examples

**EXAMPLE 1:** Dimerization of 1-Hexene: A nickel oxide dimerization catalyst for the dimerization of a C₆ olefin stream was prepared by the method described for Example 1 in U.S. Patent No. 5,169,824 to Saleh et al., entitled "Catalyst Comprising Amorphous NiO On Silica/Alumina Support". The dimerization results are tabulated in Table 1.

**Table 1**

| **Test Results** | **Example 1** |
|---|---|
| Conversion (wt.%) | 59 |
| C₁₂ olefin dimer (wt%) | 22 |
| % Conversion of branched C₁₂ olefins to total C₁₂ olefins | 77 |

**EXAMPLE 2: DIMERIZATION OF DILATED 1-HEXENE:** A nickel oxide dimerization catalyst for the dimerization of a C₆ olefin stream was prepared by the method described for Example 1 in U.S. Patent No. 5,169,824 to Saleh et al., entitled "Catalyst Comprising Amorphous NiO On Silica/Alumina Support". A 15 mL reactor tube of the autoclave unit was charged with the NiO catalyst (0.335 grams), 1-hexene (1.675 grams), hexane (1.675 grams), and a gas chromatography standard (0.67 grams linear tetradecane). The dimerization results are tabulated in Table 2.

**Table 2**

| **Test Results.** | **Example 2** |
|---|---|
| Conversion (wt.%) | 54 |
| C₁₂ olefin dimer (wt.%) | 8 |
| % Conversion of branched C₁₂ olefins to total C₁₂ olefins | 82 |

Further modifications and alternative embodiments of various aspects of the invention may be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as the presently preferred embodiments. Elements and materials may be substituted for those illustrated and described herein, parts and processes may be reversed, and certain features of the invention may be utilized independently, all as would be apparent to one skilled in the art after having the benefit of this description of the invention.

## Claims

1. A method for the production of alkyl aromatic hydrocarbons, comprising:
introducing a first hydrocarbon stream comprising olefins and paraffins, as produced from a Fischer-Tropsch process, into a dimerization unit, wherein the dimerization unit is configured to dimerize at least a portion of the olefins in the first hydrocarbon stream to produce dimerized olefins, and wherein at least a portion of the unreacted components of the first hydrocarbon stream and at least a portion of the produced dimerized olefins form a second hydrocarbon stream, and wherein at least a portion of the dimerized olefins are branched olefins; and wherein the dimerization unit comprises a dimerization catalyst configured to dimerize at least a portion of the olefins, the catalyst comprising nickel oxide; and
introducing at least a portion of the second hydrocarbon stream and aromatic hydrocarbons into an alkylation unit, wherein the alkylation unit is configured to alkylate at least a portion of the aromatic hydrocarbons with at least a portion of the olefins from the second hydrocarbon stream to produce alkyl aromatic hydrocarbons, wherein a least a portion of the produced alkyl aromatic hydrocarbons comprise a branched alkyl group, wherein
the first hydrocarbon stream comprises olefins having a carbon number from 4 to 8 or a carbon number from 4 to 6, wherein the olefin composition of the second hydrocarbon stream comprises linear and branched olefins having a carbon number from 10 to 16 and wherein at least a portion of the branched olefins comprises methyl and ethyl branches and wherein the catalyst comprising nickel oxide is an amorphous nickel oxide present as a dispersed substantial monolayer on the surfaces of a silica support, said support also including minor amounts of an oxide of aluminium, gallium or indium such that the ratio of nickel oxide to metal oxide present in the catalyst is within the range of 4:1 to 100:1 and wherein said silica support has a surface area within the range of from 100 m²/g to 450 m²/g.

2. The method of claim 1, wherein the first hydrocarbon stream is produced from a Fischer-Tropsch process.

3. The method of any one of claims 1-2, wherein the olefin composition in the first hydrocarbon stream comprises linear and branched olefins, and/or between 50 percent and 99 percent olefins.

4. The method of any one of claims 1 to 2, wherein the first hydrocarbon stream comprises between 50 percent and 99 percent olefins.

5. The method of any one of claims 1 to 3, wherein the dimerization unit is operated at temperature between 120 °C and 200 °C or between 150 °C and 165 °C.

6. The method of any one of claims 1 to 4, wherein the dimerization unit is configured to produce greater than 20 percent of a branched dimer compound.

7. The method of any one of claims 1 to 5, wherein a portion of the branched olefins comprise an average number of branches per total olefin molecules of at least 0.7, between 0.7 and 1.5 between 0.7 and 2.0, between 1.0 and 1.5, or less than 2.5.

8. The method of any one of claims 1 to 6, wherein portion of the branched olefins comprise methyl and ethyl branches.

9. The method of any one of claims 1 to 7, wherein the branched groups on the branched olefins are greater than 50 percent methyl groups; less than 10 percent ethyl groups and/or less than 5 percent groups other than methyl or ethyl groups.

10. The method of any one of claims 1 to 8, wherein the branched olefins have less than 0.5 percent aliphatic quaternary carbon atoms or less than about 0.3 percent aliphatic quaternary carbon atoms.

11. The method of any one of claims 1 to 9, further comprising:
separating the produced dimerized olefins from the second hydrocarbon stream to form a produced dimerized olefins stream and a paraffins and unreacted olefins stream, wherein the paraffins and unreacted olefins stream comprises hydrocarbons of carbon numbers less than 7; and
introducing at least a portion of the paraffins and unreacted olefins stream into the dimerization unit.

12. The method of any one of claims 1 to 10, further comprising adjusting a ratio of olefins to paraffins introduced into the alkylation unit by adding at least a portion of a third hydrocarbon stream into the alkylation unit, or by adding at least a portion of a third hydrocarbon stream into the alkylation unit wherein the third hydrocarbon stream comprises greater than 90 percent paraffins by weight.

13. The method of any one of claims 1 to 11, further comprising:
adjusting a ratio of olefins to paraffins introduced into the alkylation unit by combining at least a portion of a third hydrocarbon stream with at least a portion of the second hydrocarbon stream upstream of the alkylation unit to form a combined stream; by combining at least a portion of a third hydrocarbon stream with at least a portion of the second hydrocarbon stream upstream of the alkylation unit to form a combined stream, wherein the third hydrocarbon stream comprises greater than about 90 percent paraffins by weight; or by combining at least a portion of a third hydrocarbon stream with at least a portion of the second hydrocarbon stream upstream of the alkylation unit to form a combined stream, wherein the third hydrocarbon stream comprises paraffins and olefins having a carbon number from 10 to 16; and
introducing the combined stream into the alkylation unit.

14. The method of any one of claims 1 to 12, wherein the alkylation unit is configured to produce greater than 50 percent or greater than 85 percent of monoalkylated aromatic hydrocarbons.

15. The method of any one of claims 1 to 13, wherein a molar ratio of the aromatic hydrocarbons to the branched olefins is between 0.1 and 2.0 in the alkylation unit.

16. The method of any one of claims 1 to 14, wherein the alkylation unit is operated at a reaction temperature between 30 °C and 300 °C.

17. The method of any one of claims 1 to 15, wherein the branched alkyl groups of the alkyl aromatic hydrocarbons comprise 0.5 percent or less aliphatic quaternary carbon atoms, and an average number of branches per alkyl group of at least 0.7, the branches comprising methyl and ethyl branches.

18. The method of any one of claim 1 to 16, further comprising:
forming an alkylation reaction stream wherein the alkylation reaction stream comprises at least a portion of the aromatic hydrocarbons, at least a portion of the unreacted components of the second hydrocarbon stream and at least a portion of the produced alkyl aromatic hydrocarbons;
separating alkyl aromatic hydrocarbons from the alkylation reaction stream to produce an unreacted hydrocarbons stream and an alkyl aromatic hydrocarbon stream; the unreacted hydrocarbons stream comprising at least a portion of the unreacted components of the second hydrocarbon stream and at least a portion of the aromatic hydrocarbons;
separating at least a portion of the paraffins and at least a portion of the olefins from the unreacted hydrocarbons stream to produce an aromatic hydrocarbons stream and a paraffins and unreacted olefins stream;
introducing at least a portion of the aromatic hydrocarbons stream into the alkylation unit.

19. The method of claim 18, further comprising separating olefins from the paraffins and unreacted olefins stream to produce an olefinic stream, wherein the olefins in the olefinic stream have carbon numbers from 4 to 8 and introducing at least a portion of the olefinic stream into the dimerization unit.

20. The method of any one of claims 1 to 18, further comprising introducing at least a portion of the alkyl aromatic hydrocarbons stream into a sulfonation unit, wherein the sulfonation unit is configured to sulfonate at least a portion of the alkyl aromatic hydrocarbons in the alkyl aromatic hydrocarbons stream to produce alkyl aromatic sulfonates; and wherein at least a portion of the alkyl aromatic sulfonates produced comprise branched alkyl aromatic sulfonates.

## Patentansprüche

1. Verfahren zur Herstellung von alkylaromatischen Kohlenwasserstoffen, umfassend:
das Einführen eines ersten Kohlenwasserstoffstroms, wie er in einem Fischer-Tropsch-Verfahren hergestellt wird, welcher Olefine und Paraffine umfasst, in eine Dimerisierungseinheit, wobei die Dimerisierungseinheit konfiguriert ist, um wenigstens einen Teil der Olefine in dem ersten Kohlenwasserstoffstrom zu dimerisieren, um dimerisierte Olefine herzustellen, und wobei wenigstens ein Teil der nicht umgesetzten Komponenten des ersten Kohlenwasserstoffstroms und wenigstens ein Teil der hergestellten dimerisierten Olefine einen zweiten Kohlenwasserstoffstrom ausbilden, und wobei wenigstens ein Teil der dimerisierten Olefine verzweigte Olefine sind; und wobei die Dimerisierungseinheit einen Dimerisierungskatalysator umfasst, der konfiguriert ist, um wenigstens einen Teil der Olefine zu dimerisieren und der Katalysator Nickeloxid umfasst; und
das Einführen von wenigstens einem Teil des zweiten Kohlenwasserstoffstroms und von aromatischen Kohlenwasserstoffen in eine Alkylierungseinheit, wobei die Alkylierungseinheit konfiguriert ist, um wenigstens einen Teil der aromatischen Kohlenwasserstoffe mit wenigstens einem Teil der Olefine aus dem zweiten Kohlenwasserstoffstrom zu alkylieren, um alkylaromatische Kohlenwasserstoffe herzustellen, wobei wenigstens ein Teil der hergestellten alkylaromatischen Kohlenwasserstoffe eine verzweigte Alkylgruppe umfasst, wobei
der erste Kohlenwasserstoffstrom Olefine mit einer Kohlenstoffzahl von 4 bis 8 oder einer Kohlenstoffzahl von 4 bis 6 umfasst, wobei die Olefinzusammensetzung des zweiten Kohlenwasserstoffstroms lineare und verzweigte Olefine mit einer Kohlenstoffzahl von 10 bis 16 umfasst, und wobei wenigstens ein Teil der verzweigten Olefine Methyl- und Ethylverzweigungen umfasst, und wobei der Nickeloxid umfassende Katalysator ein amorphes Nickeloxid ist, das als substantielle Monoschicht auf der Oberfläche eines Siliciumoxidträgers dispergiert ist, welcher Träger auch geringe Mengen an Oxiden von Aluminium, Gallium oder Indium einschließt, sodass das Verhältnis von Nickeloxid zu Metalloxid, welches im Katalysator vorhanden ist, im Bereich von 4:1 bis 100:1 beträgt, und wobei der Siliciumoxidträger eine Oberfläche im Bereich von 100 m²/g bis 450 m²/g besitzt.

2. Verfahren nach Anspruch 1, wobei der erste Kohlenwasserstoffstrom aus einem Fischer-Tropsch-Verfahren stammt.

3. Verfahren nach einem der Ansprüche 1 bis 2, wobei die Olefinzusammensetzung im ersten Kohlenwasserstoffstrom lineare und verzweigte Olefine, und/oder von 50 Prozent bis 99 Prozent Olefine, umfasst.

4. Verfahren nach einem der Ansprüche 1 bis 2, wobei der erste Kohlenwasserstoffstrom von 50 Prozent bis 99 Prozent Olefine umfasst.

5. Verfahren nach einem der Ansprüche 1 bis 3, wobei die Dimerisierungseinheit bei einer Temperatur von 120°C bis 200°C oder von 150°C bis 165°C betrieben wird.

6. Verfahren nach einem der Ansprüche 1 bis 4, wobei die Dimerisierungseinheit konfiguriert ist, um mehr als 20 Prozent von einer verzweigten Dimerverbindung herzustellen.

7. Verfahren nach einem der Ansprüche 1 bis 5, wobei ein Teil der verzweigten Olefine eine durchschnittliche Zahl an Verzweigungen pro Gesamtolefinmolekülen von wenigstens 0,7, von 0,7 bis 1,5, von 0,7 bis 2,0, von 1,0 bis 1,5, oder weniger als 2,5 umfasst.

8. Verfahren nach einem der Ansprüche 1 bis 6, wobei ein Teil der verzweigten Olefine Methyl- und Ethylverzweigungen umfasst.

9. Verfahren nach einem der Ansprüche 1 bis 7, wobei die verzweigten Gruppen an den verzweigten Olefinen mehr als 50 Prozent Methylgruppen, weniger als 10 Prozent Ethylgruppen, und/oder weniger als 5 Prozent andere Gruppen als Methyl- oder Ethylgruppen sind.

10. Verfahren nach einem der Ansprüche 1 bis 8, wobei die verzweigten Olefine weniger als 0,5 Prozent aliphatische quaternäre Kohlenstoffatome oder weniger als 0,3 Prozent aliphatische quaternäre Kohlenstoffatome besitzen.

11. Verfahren nach einem der Ansprüche 1 bis 9, ferner umfassend:
das Abtrennen der hergestellten dimerisierten Olefine aus dem zweiten Kohlenwasserstoffstrom, um einen Strom aus dimerisierten Olefinen und einen Strom aus Paraffinen und nicht umgesetzten Olefinen auszubilden, wobei der Strom aus Paraffinen und nicht umgesetzten Olefinen Kohlenwasserstoffe mit Kohlenstoffzahlen von weniger als 7 umfasst; und
das Einführen von wenigstens einem Teil des Stroms aus Paraffinen und nicht umgesetzten Olefinen in die Dimerisierungseinheit.

12. Verfahren nach einem der Ansprüche 1 bis 10, ferner umfassend das Einstellen eines Verhältnisses von Olefinen zu Paraffinen, welche in die Alkylierungseinheit eingeführt werden, durch Zufügen von wenigstens einem Teil eines dritten Kohlenwasserstoffstroms in die Alkylierungseinheit, oder durch Zufügen von wenigstens einem Teil eines dritten Kohlenwasserstoffstroms in die Alkylierungseinheit, wobei der dritte Kohlenwasserstoffstrom mehr als 90 Gew.-% Paraffine umfasst.

13. Verfahren nach einem der Ansprüche 1 bis 11, ferner umfassend:
das Einstellen eines Verhältnisses von Olefinen zu Paraffinen, welche in die Alkylierungseinheit eingeführt werden, durch Kombinieren von wenigstens einem Teil eines dritten Kohlenwasserstoffstroms, stromaufwärts zur Alkylierungseinheit, mit wenigstens einem Teil des zweiten Kohlenwasserstoffstroms, um einen kombinierten Strom zu bilden; durch Kombinieren von wenigstens einem Teil eines dritten Kohlenwasserstoffstroms, stromaufwärts zur Alkylierungseinheit, mit wenigstens einem Teil des zweiten Kohlenwasserstoffstroms, um einen kombinierten Strom auszubilden, wobei der dritte Kohlenwasserstoffstrom mehr als etwa 90 Gew.-% Paraffine umfasst; oder durch Kombinieren von wenigstens einem Teil eines dritten Kohlenwasserstoffstroms, stromaufwärts zur Alkylierungseinheit, mit wenigstens einem Teil des zweiten Kohlenwasserstoffstroms, um einen kombinierten Strom auszubilden, wobei der dritte Kohlenwasserstoffstrom Paraffine und Olefine mit einer Kohlenstoffzahl von 10 bis 16 umfasst; und
das Einführen des kombinierten Stroms in die Alkylierungseinheit.

14. Verfahren nach einem der Ansprüche 1 bis 12, wobei die Alkylierungseinheit konfiguriert ist, um mehr als 50 Prozent oder mehr als 85 Prozent monoalkylierte aromatische Kohlenwasserstoffe herzustellen.

15. Verfahren nach einem der Ansprüche 1 bis 13, wobei ein Molverhältnis von den aromatischen Kohlenwasserstoffen zu den verzweigten Olefinen in der Alkylierungseinheit von 0,1 bis 2,0 vorliegt.

16. Verfahren nach einem der Ansprüche 1 bis 14, wobei die Alkylierungseinheit bei einer Reaktionstemperatur von 30°C bis 300°C betrieben wird.

17. Verfahren nach einem der Ansprüche 1 bis 15, wobei die verzweigten Alkylgruppen der alkylaromatischen Kohlenwasserstoffe 0,5 Prozent oder weniger aliphatische quaternäre Kohlenstoffatome und eine durchschnittliche Anzahl an Verzweigungen pro Alkylgruppe von wenigstens 0,7 umfassen, und die Verzweigungen Methyl- und Ethylverzweigungen umfassen.

18. Verfahren nach einem der Ansprüche 1 bis 16, ferner umfassend:
das Bilden eines Alkylierungsreaktionsstromes, wobei der Alkylierungsreaktionsstrom wenigstens einen Teil der aromatischen Kohlenwasserstoffe, wenigstens einen Teil der nicht umgesetzten Komponenten des zweiten Kohlenwasserstoffstroms, und wenigstens einen Teil der hergestellten alkylaromatischen Kohlenwasserstoffe umfasst;
das Abtrennen der alkylaromatischen Kohlenwasserstoffe aus dem Alkylierungsreaktionsstrom, um einen Strom an nicht umgesetzten Kohlenwasserstoffen und einen alkylaromatischen Kohlenwasserstoffstrom auszubilden, wobei der Strom an nicht umgesetzten Kohlenwasserstoffen wenigstens einen Teil der nicht umgesetzten Komponenten des zweiten Kohlenwasserstoffstroms und wenigstens einen Teil der aromatischen Kohlenwasserstoffe umfasst;
das Abtrennen von wenigstens einem Teil der Paraffine und wenigstens einem Teil der Olefine aus dem Strom aus nicht umgesetzten Kohlenwasserstoffen, um einen aromatischen Kohlenwasserstoffstrom und einen Strom aus Paraffinen und nicht umgesetzten Olefinen auszubilden;
das Einführen von wenigstens einem Teil des aromatischen Kohlenwasserstoffstroms in die Alkylierungseinheit.

19. Verfahren nach Anspruch 18, ferner umfassend das Abtrennen von Olefinen aus dem Strom aus Paraffinen und nicht umgesetzten Olefinen, um einen olefinischen Strom auszubilden, wobei die Olefine im olefinischen Strom eine Kohlenstoffzahl von 4 bis 8 besitzen, und das Einführen von wenigstens einem Teil des olefinischen Stroms in die Dimerisierungseinheit.

20. Verfahren nach einem der Ansprüche 1 bis 18, ferner umfassend das Einführen von wenigstens einem Teil des alkylaromatischen Kohlenwasserstoffstroms in eine Sulfonierungseinheit, wobei die Sulfonierungseinheit konfiguriert ist, um wenigstens einen Teil der alkylaromatischen Kohlenwasserstoffe in dem alkylaromatischen Kohlenwasserstoffstrom zu sulfonieren, um alkylaromatische Sulfonate auszubilden; und wobei wenigstens ein Teil der hergestellten alkylaromatischen Sulfonate verzweigte alkylaromatische Sulfonate umfasst.

## Revendications

1. Procédé pour la production d'hydrocarbures alkylaromatiques, comprenant :
l'introduction d'un premier courant d'hydrocarbures comprenant des oléfines et des paraffines, telles que produites par un procédé de Fischer-Tropsch, dans une unité de dimérisation, dans lequel l'unité de dimérisation est configurée pour dimériser au moins une partie des oléfines dans le premier courant d'hydrocarbures afin de produire des oléfines dimérisées, dans lequel au moins une partie des composants n'ayant pas réagi du premier courant d'hydrocarbures et au moins une partie des oléfines dimérisées produites forment un deuxième courant d'hydrocarbures et dans lequel au moins une partie des oléfines dimérisées est formée d'oléfines ramifiées ; et dans lequel l'unité de dimérisation comprend un catalyseur de dimérisation configuré pour dimériser au moins une partie des oléfines, le catalyseur comprenant de l'oxyde de nickel ; et
l'introduction d'au moins une partie du deuxième courant d'hydrocarbures et d'hydrocarbures aromatiques dans une unité d'alkylation, dans lequel l'unité d'alkylation est configurée pour alkyler au moins une partie des hydrocarbures aromatiques avec au moins une partie des oléfines provenant du deuxième courant d'hydrocarbures pour produire des hydrocarbures alkylaromatiques, dans lequel au moins une partie des hydrocarbures alkylaromatiques comprend un groupement alkyle ramifié, dans lequel le premier courant d'hydrocarbures comprend des oléfines ayant un nombre de carbone de 4 à 8 ou un nombre de carbone de 4 à 6, dans lequel la composition oléfinique du deuxième courant d'hydrocarbures comprend des oléfines linéaires et ramifiées ayant un nombre de carbone de 10 à 16, dans lequel au moins une partie des oléfines ramifiées comprend des ramifications méthyle et éthyle et dans lequel le catalyseur comprenant de l'oxyde de nickel est un oxyde de nickel amorphe présent sous la forme d'une monocouche substantielle dispersée sur les surfaces d'un support de silice, ledit support comprenant également des quantités mineures d'un oxyde d'aluminium, de gallium ou d'indium de sorte que le rapport de l'oxyde de nickel à l'oxyde de métal présent dans le catalyseur se situe dans la plage de 4:1 à 100:1 et dans lequel ledit support de silice a une surface spécifique dans la plage de 100 m²/g à 450 m²/g.

2. Procédé selon la revendication 1, dans lequel le premier courant d'hydrocarbures est produit par un procédé de Fischer-Tropsch.

3. Procédé selon l'une quelconque des revendications 1 et 2, dans lequel la composition oléfinique dans le premier courant d'hydrocarbures comprend des oléfines linéaires et ramifiées et/ou 50 pour-cent à 99 pour-cent d'oléfines.

4. Procédé selon l'une quelconque des revendications 1 à 2, dans lequel le premier courant d'hydrocarbures comprend 50 pour-cent à 99 pour-cent d'oléfines.

5. Procédé selon l'une quelconque des revendications 1 à 3, dans lequel l'unité de dimérisation est activée à une température de 120 °C à 200 °C ou de 150 °C à 165 °C.

6. Procédé selon l'une quelconque des revendications 1 à 4, dans lequel l'unité de dimérisation est configurée pour produire plus de 20 pour-cent d'un composé dimère ramifié.

7. Procédé selon l'une quelconque des revendications 1 à 5, dans lequel une partie des oléfines ramifiées comprend un nombre moyen de ramifications par total de molécules d'oléfines d'au moins 0,7, de 0,7 à 1,5, de 0,7 à 2,0, de 1,0 à 1,5 ou de moins de 2,5.

8. Procédé selon l'une quelconque des revendications 1 à 6, dans lequel une partie des oléfines ramifiées comprend des ramifications méthyle et éthyle.

9. Procédé selon l'une quelconque des revendications 1 à 7, dans lequel les groupements ramifiés sur les oléfines ramifiées sont de plus de 50 pour-cent de groupements méthyle ; de moins de 10 pour-cent des groupements éthyle et/ou de moins de 5 pour-cent de groupements autres que méthyle ou éthyle.

10. Procédé selon l'une quelconque des revendications 1 à 8, dans lequel les oléfines ramifiées ont moins de 0,5 pour-cent d'atomes de carbone quaternaires aliphatiques ou moins d'environ 0,3 pour-cent d'atomes de carbone quaternaires aliphatiques.

11. Procédé selon l'une quelconque des revendications 1 à 9, comprenant en outre :
la séparation des oléfines dimérisées produites du deuxième courant d'hydrocarbures pour former un courant d'oléfines dimérisées et un courant de paraffines et d'oléfines n'ayant pas réagi, dans lequel le courant de paraffines et d'oléfines n'ayant pas réagi comprend des hydrocarbures ayant des nombres de carbone inférieurs à 7 ; et
l'introduction d'au moins une partie du courant de paraffines et d'oléfines n'ayant pas réagi dans l'unité de dimérisation.

12. Procédé selon l'une quelconque des revendications 1 à 10, comprenant en outre l'ajustement d'un rapport des oléfines aux paraffines introduit dans l'unité d'alkylation en ajoutant au moins une partie d'un troisième courant d'hydrocarbures dans l'unité d'alkylation ou en ajoutant au moins une partie d'un troisième courant d'hydrocarbures dans l'unité d'alkylation, dans lequel le troisième courant d'hydrocarbures comprend plus de 90 pour-cent de paraffines en poids.

13. Procédé selon l'une quelconque des revendications 1 à 11, comprenant en outre :
l'ajustement d'un rapport des oléfines aux paraffines introduites dans l'unité d'alkylation en combinant au moins une partie d'un troisième courant d'hydrocarbures avec au moins une partie du deuxième courant d'hydrocarbures en amont de l'unité d'alkylation pour former un courant combiné ; en combinant au moins une partie du troisième courant d'hydrocarbures avec au moins une partie du deuxième courant d'hydrocarbures en amont de l'unité d'alkylation pour former un courant combiné, dans lequel le troisième courant d'hydrocarbures comprend plus d'environ 90 pour-cent de paraffines en poids ; ou en combinant au moins une partie d'un troisième courant d'hydrocarbures avec au moins une partie du deuxième courant d'hydrocarbures en amont de l'unité d'alkylation pour former un courant combiné, dans lequel le troisième courant d'hydrocarbures comprend des paraffines et des oléfines ayant un nombre de carbone de 10 à 16 ; et
l'introduction du courant combiné dans l'unité d'alkylation.

14. Procédé selon l'une quelconque des revendications 1 à 12, dans lequel l'unité d'alkylation est configuré pour produire plus de 50 pour-cent ou plus de 85 pour-cent d'hydrocarbures aromatiques monoalkyés.

15. Procédé selon l'une quelconque des revendications 1 à 13, dans lequel un rapport molaire des hydrocarbures aromatiques aux oléfines ramifiées est de 0,1 à 2,0 dans l'unité d'alkylation.

16. Procédé selon l'une quelconque des revendications 1 à 14, dans lequel l'unité d'alkylation est exploitée à une température réactionnelle de 300 °C à 300 °C.

17. Procédé selon l'une quelconque des revendications 1 à 15, dans lequel les groupements alkyle ramifiés des hydrocarbures alkylaromatiques comprennent 0,5 pour-cent ou moins d'atomes de carbone quaternaires aliphatiques et un nombre moyen de ramifications par groupement alkyle d'au moins 0,7, les ramifications comprenant des ramifications méthyle et éthyle.

18. Procédé selon l'une quelconque des revendications 1 à 16, comprenant en outre :
la formation d'un courant de réaction d'alkylation dans lequel le courant de réaction d'alkylation comprend au moins une partie des hydrocarbures aromatiques, au moins une partie des composants n'ayant pas réagi du deuxième courant d'hydrocarbures et au moins une partie des hydrocarbures alkylaromatiques produits ;
la séparation d'hydrocarbures alkyl-aromatiques du courant de réaction d'alkylation pour produire un courant d'hydrocarbures n'ayant pas réagi et un courant d'hydrocarbures alkylaromatiques ; le courant d'hydrocarbures n'ayant pas réagi comprenant au moins une partie des composants n'ayant pas réagi du deuxième courant d'hydrocarbures et au moins une partie des hydrocarbures aromatiques ;
la séparation d'au moins une partie des paraffines et d'au moins une partie des oléfines du courant d'hydrocarbures n'ayant pas réagi pour produire un courant d'hydrocarbures aromatiques et un courant de paraffines et d'oléfines n'ayant pas réagi ;
l'introduction d'au moins une partie du courant d'hydrocarbures aromatiques dans l'unité d'alkylation.

19. Procédé selon la revendication 18, comprenant en outre la séparation d'oléfines du courant de paraffines et d'oléfines n'ayant pas réagi pour produire un courant oléfinique, dans lequel les oléfines du courant oléfinique ont des nombres de carbone de 4 à 8 et l'introduction d'au moins une partie du courant oléfinique dans l'unité de dimérisation.

20. Procédé selon l'une quelconque des revendications 1 à 18, comprenant en outre l'introduction d'au moins une partie du courant d'hydrocarbures alkylaromatiques dans une unité de sulfonation, dans lequel l'unité de sulfonation est configurée pour sulfoner au moins une partie des hydrocarbures alkylaromatiques dans le courant d'hydrocarbures alkylaromatiques afin de produire des sulfonates alkylaromatiques ; et dans lequel au moins une partie des sulfonates alkylaromatiques produits comprend des sulfonates alkylaromatiques ramifiés.
